# EUROPÄISCHE PATENTANMELDUNG

(11) **EP 0 646 785 A1**
(43) Veröffentlichungstag der Anmeldung: **05.04.1995**
(21) Anmeldenummer: 94115510.3
(22) Anmeldetag: 30.09.1994
(51) Int. Cl.: G01N 22/04, G01N 33/28

(54) **Verfahren und Anordnung zur kontinuierlichen Messung des Wassergehaltes eines Fluides**

(30) Priorität: 01.10.1993 DE 4333540
(71) Anmelder: LVE VERFAHRENSELEKTRONIK GMBH, D-45138 Essen (DE)
(72) Erfinder: Maiwald, Detlef, D-47447 Moers (DE); Nolte, Karl, Dr., D-45259 Essen (DE)
(74) Vertreter: Harlacher, Mechthild

(57) **Zusammenfassung**

Zur Messung des Wassergehaltes eines Fluides, insbesondere von Öl in Ölbädern von Wärmebehandlungsanlagen, wird kontinuierlich ein Teilstrom des Fluides durch eine Meßzelle (2) in Form eines metallischen Hohlleiters geleitet, während Mikrowellen in den Hohlleiter eingekoppelt und ausgekoppelt werden. Die Mikrowellen werden durch den Hohlleiter geleitet. Das von einem Detektor (10) empfangene Signal stellt ein Maß für die Dämpfung dar. In einer Auswerte- und Steuereinheit (11) wird das analoge Signal in an sich bekannter Art und Weise digitalisiert und kann angezeigt werden.

## Beschreibung

Die Erfindung betrifft ein Verfahren und eine Anordnung zur kontinuierlichen Messung des Wassergehaltes eines Fluides, insbesondere von Öl in Ölbädern von Wärmebehandlungsanlagen, wobei von einem Sender erzeugte Mikrowellen durch das Fluid geleitet und von einem Detektor empfangen werden und wobei der Detektor ein zum Wassergehalt proportionales, analoges Signal abgibt, welches in einer nachgeschalteten Auswerteeinheit verarbeitet wird.

Aus der EP-B1 0032061 ist ein derartiges Verfahren und eine Vorrichtung zur Messung des Wasserprozentgehaltes von in einer Leitung fließenden Rohöles bekannt. Über Hornstrahler werden Mikrowellen quer durch den Rohölstrom geleitet und von einem Detektor aufgefangen. Zusätzlich kreuzen Gammastrahlen den Ölstrom und werden von einem zweiten Detektor empfangen. Dieser gibt ein Signal ab, das mit dem Signal aus dem ersten Detektor zusammen verarbeitet wird. Es handelt sich hier um ein großtechnisches, sehr aufwendiges Verfahren.

Öl, insbesondere Härte-, Warmbad oder Anlaßöl in Wärmebehandlungsanlagen darf kein Wasser enthalten. Insbesondere in Härteölbädern ist unerkanntes Wasser sehr problematisch, denn es führt bei Abschreckvorgängen zu erheblicher Schaumbildung. Dadurch können explosionsartige Ölbrände entstehen.

In Abschrecköl führen bereits geringe Wassergehalte zu Weichfleckigkeit an den gehärteten Teilen. Die Folge sind schlechte Prozeßergebnisse in Form von verstärktem Härteverzug, und in Extremfällen in Form von Rußbildung an den gehärteten Teilen.

Aus der Praxis ist ein kontinuierliches Verfahren auf Infrarotbasis bekannt, das allerdings gegenüber Verschmutzungen im Öl empfindlich ist.

Weitverbreitet in der Praxis sind in der Regel diskontinuierliche Verfahren, bei denen das Öl erhitzt wird, wobei eventuelle Wasseranteile verdampfen und ein Warnsignal erzeugen. Diese Verfahran sind apparatetechnisch aufwendig und wartungsintensiv.

Der Erfindung liegt daher die Aufgabe zugrunde, ein Verfahren und eine Vorrichtung der eingangs genannten Art zu vereinfachen, um einen wirtschaftlichen Einsatz zur Messung des Wassergehaltes eines Fluides, insbesondere von Öl in Ölbädern, zu ermöglichen.

Zur Lösung dieser Aufgabe ist das Verfahren nach der Erfindung dadurch gekennzeichnet, daß kontinuierlich ein Teilstrom des Fluides durch eine Meßzelle in Form eines metallischen Hohlleiters geleitet wird und daß die Mikrowellen in den Hohlleiter eingekoppelt und ausgekoppelt werden.

Es wird eine Dämpfungsmessung durchgeführt. Das Meßverfahren beruht auf der wassergehaltsabhängigen Dämpfung der Mikrowellen. Dabei wird ausgenutzt, daß die komplexe Dielektrizitätszahl von beispielsweise Öl niedrig ist und Wasseranteile die Dämpfung erheblich erhöhen.

Die Mikrowellen werden durch den Hohlleiter geführt. Das vom Detektor empfangene Signal stellt ein Maß für die Dämpfung dar. In der Auswerteeinheit wird das analoge Signal in an sich bekannter Art und Weise digitalisiert und kann angezeigt werden.

Das Verfahren ermöglicht eine einfache kontinuierliche Überwachung. Es findet keine Beeinflussung des Fluides statt. Insbesondere wird das Fluid nicht thermisch belastet. Besonders vorteilhaft ist die hohe Zuverlässigkeit auch bei veränderlichen physikalischen Eigenschaften des Fluides. Das Verfahren ist insbesondere für die kontinuierliche Prüfung von Öl in Ölbädern geeignet, weil es ölsortenunabhängig, verschmutzungsunempfindlich und wartungsfrei ist.

Vorteilhaft ist auch die hohe Ansprechempfindlichkeit. Mit dem Verfahren ist es möglich, Wasserkonzentrationen von weniger als 1 % im Fluid sicher nachzuweisen.

Da es sich um einen geschlossenen Mikrowellen-Meßkreis handelt, können Mikrowellen nicht in die Umgebung abgestrahlt werden, wodurch Schäden vermieden werden.

Vorteilhafterweise wird die Frequenz der Mikrowellen im Bereich von 8,0 bis 24 GHz vorzugsweise 10 bis 13 GHz eingestellt.

Die monofrequente Dämpfungsmessung ist besonders einfach. Die Frequenz wird nur in einem schmalbandigen Bereich geändert, und zwar um Verfälschungen durch Welligkeiten zu vermeiden. In der Auswerteeinheit wird in an sich bekannter Art und Weise der Mittelwert über die Einzelfrequenzen gebildet.

Eine wesentliche Weiterbildung des Verfahrens besteht darin, daß Mikrowellen zusätzlich in eine Referenzmeßzelle ein- bzw. ausgekoppelt werden. Schwankungen der Meßelektronik, beispielsweise Frequenz oder Amplitudenschwankungen des Mikrowellensenders oder Alterungseffekte im Dauerbetrieb, können auf diese Weise ausgeschaltet werden. Durch Verhältnisbildung der beiden Detektorsignale entfallen Kurz- und Langzeitinstabilitäten des Mikrowellensenders, die sonst voll in die Messung eingehen sowie die Genauigkeit und die Auflösung verschlechtern können.

Wenn die Temperatur des Fluides schwankt, ist es vorteilhaft, die Temperatur des Fluides zu messen und den Meßwert zur Korrektur des zum Wassergehalt proportionalen Signals zu verwenden.

Das erfindungsgemäße Gerät zur kontinuierlichen Messung des Wassergehaltes eines Fluides, insbesondere von Öl in Ölbädern von Wärmebehandlungsanlagen hat ebenso wie die bekannte Vorrichtung gleicher Gattung eine Meßzelle, einen Mikrowellensender und einen Detektor, der mit einer Auswerteeinheit verbunden ist. Das erfindungsgemäße Gerät ist zur Durchführung des zuvor beschriebenen Meßverfahrens besonders geeignet. Es zeichnet sich dadurch aus, daß die Meßzelle einen Meßraum aufweist, der mit mindestens einer Eintrittsöffnung und mindestens einer Austrittsöffnung für das Fluid versehen ist und daß sich an den Meßraum stirnseitig je ein luftgefüllter Raum anschließt, in den über je einen Stiftkoppler die Mikrowellen ein- bzw. ausgekoppelt werden.

Die Eintritts- bzw. die Austrittsöffnung ist in der Nähe der Enden der Meßzelle angeordnet und mit je einem Leitungsstutzen versehen, der an eine Fluid-Hauptleitung anschließbar ist.

Der apparative Aufwand des Gerätes ist gering und dementsprechend sind die Kosten niedrig. Der Elektronikteil besteht aus üblichen Komponenten ohne besondere Eigenschaffen, z. B hochstabile Bausteine. Das Meßgerät kann außerdem sehr kompakt und handlich aufgebaut werden.

Vorzugsweise ist die Auswerteeinheit mit einer Alarmeinrichtung versehen, die ein Alarmsignal erzeugt, wenn Wasser im Fluid vorhanden ist.

Vorteilhafterweise ist der Hohlleiter als im wesentlichen in sich geschlossener Rechteckhohlleiter ausgebildet. Die Verwendung eines Hohlleiters mit rundem Querschnitt ist ebenfalls möglich. Allerdings sind derartige Hohlleiter kostenaufwendiger.

Nach einem vorteilhaften Merkmal der Erfindung ist die Eintrittsöffnung und die Austrittsöffnung für das Fluid an der breiten Seite des Rechteckhohlleiters angeordnet, um den Feldverlauf nicht zu stören.

Nach einem weiteren vorteilhaften Merkmal der Erfindung ist das Gerät dadurch gekennzeichnet, daß zwischen dem Meßraum und dem luftgefüllten Raum ein Anpaßelement angeordnet ist. Letzteres dient zur Anpassung des vollständig mit dem Fluid gefüllten Meßraumes an die luftgefüllten Räume, in die die Mikrowellen ein- bzw. ausgekoppelt werden.

Vorteilhafterweise ist das Anpaßelement aus einem Material mit geringer Dielektrizitätskonstante vorzugsweise Kunststoff. Beispielsweise kann Acrylglas verwendet werden.

Vorzugsweise ist das Anpaßelement keilförmig. Die Anpassung sollte in der Breitseite des Hohlleiters und möglichst in beide Richtungen (Luft/Fluid) erfolgen.

Eine wesentliche Weiterbildung der Erfindung besteht darin, daß der Mikrowellensender als spannungsgesteuerter Osszilator ausgebildet ist, der von der Auswerte- und Steuereinheit gesteuert wird. Durch Abstimmung über einen schmalen Frequenzbereich wird vermieden, daß die Messung durch Welligkeit verfälscht wird. Die erforderliche Abstimmbandbreite richtet sich nach der zu erwartenden Welligkeit. Zusätzlich werden die Mikrowellen mit einem niederfrequenten Signal zur Eliminierung von Gleichspannungen amplitudenmoduliert.

Eine weitere Weiterbildung der Erfindung besteht darin, daß der Mikrowellensender mit einem Leistungsteiler und der Detektor mit einem Mikrowellenschalter versehen ist, daß über weitere Stiftkoppler ein Teil der Mikrowellenleistung in eine Referenzmeßzelle ein- bzw. ausgekoppelt wird und daß die Referenzmeßzelle analog zur Meßzelle als metallischer Hohlleiter ausgebildet ist. Der Hohlleiter kann Luft enthalten. Es ist jedoch vorteilhaft, den Hohlleiter mit Öl zu füllen.

Alternativ dazu kann der Mikrowellensender mit einem Leistungsschalter versehen sein, ein Teil der Mikrowellenleistung zu einem Referenzdetektor geführt und das Referenzsignal aus dem Referendetektor zur Auswerte- und Steuereinheit geführt werden.

Eine bevorzugte Ausführungsform ist gekennzeichnet durch ein Gehäuse, das mittels einer Trennwand in zwei Räume geteilt ist, wobei in einem ersten Raum die Meßzelle und in einem zweiten Raum die elektronischen Bauteile montiert sind.

Das erfindungsgemäße Verfahren und das Gerät werden anhand von drei bevorzugten Ausführungsbeispielen erläutert. Die Ausführungsbeispiele beinhalten besonders geeignete Geräte zur Ermittlung von unerwünschten Wasseranteilen um Öl eines Ölbades zum Abschrecken von Härtegut.

In der Zeichnung zeigt
- Fig. 1: eine schematische Darstellung eines erfindungsgemäßen Gerätes nach einer ersten Ausführungsform;
- Fig. 2: eine schematische Darstellung des erfindungsgemäßen Gerätes nach einer zweiten Ausführungsform;
- Fig. 3: eine schematische Darstellung des erfindungsgemäßen Gerätes nach einer dritten Ausführungsform.

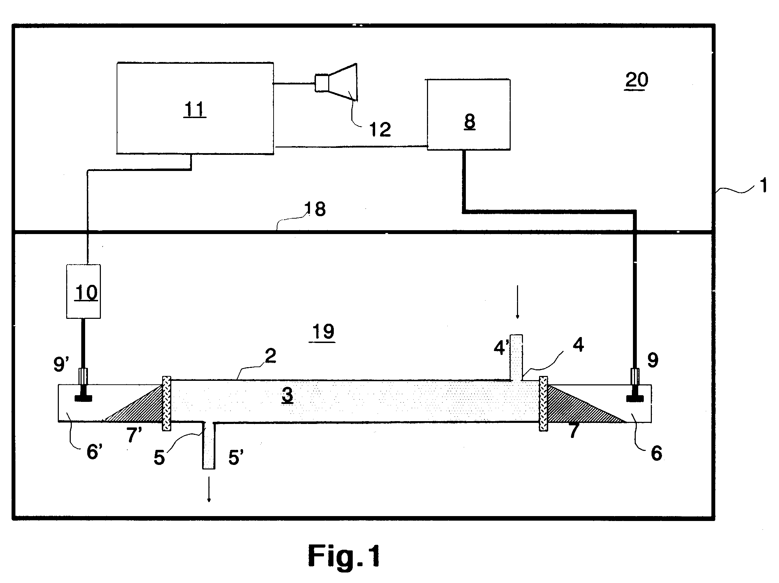

In Fig. 1 ist in einem Gehäuse 1 ist eine Meßzelle 2 in Form eines metallischen Hohlleiters mit rechteckigem Querschnitt angeordnet. Die Meßzelle 2 beinhaltet einen Meßraum 3. An einem Ende befindet sich eine Eintrittsöffnung 4, an die ein Eintrittsleitungsstutzen 4' angeschlossen ist. In der Nähe des anderen Endes befindet sich eine Austrittsöffnung 5 mit einem Leitungsstutzen 5'. Beide Leitungsstutzen 4' und 5' sind an einen nicht dargestellten Bypass eines Ölbadkreislaufes angeschlossen. Die Eintrittsöffnung 4 und die Austrittsöffnung 5 sind an der breiten Seite des Rechteckhohlleiters angeordnet, um den Feldverlauf nicht zu stören.

An den Meßraum 3 schließen sich je ein luftgefüllter Raum 6,6' an. Zwischen den luftgefüllten Räumen 6,6' und dem ölgefüllten Meßraum 3 ist je ein keilförmiges Anpaßelement 7,7' angeordnet. Die Anpassung erfolgt in der Breitseite des Hohlleiters. Sie sollte möglichst in beide Richtungen (Luft/Öl) erfolgen. Das Anpaßelement besteht aus einem Material mit geringer Dielektrizitätskonstante, vorzugsweise Kunststoff.

Ein Mikrowellensender 8 erzeugt Mikrowellen, die über einen Stiftkoppler 9 in den luftgefüllten Raum 6 eingekoppelt werden. Die Mikrowellen breiten sich in dem mit Öl gefüllten Meßraum 3 aus. Es wird eine Dämpfungsmessung durchgeführt. Ein Detektor 10 empfängt über einen weiteren Stiftkoppler 9' ein Signal, das ein Maß für die Dämpfung darstellt, und zwar in Abhängigkeit von dem Wassergehalt. Das Signal vom Detektor wird in an sich bekannter Weise in einem in einer Auswerte- und Steuereinheit 11 integrierten, nicht dargestellten Analog/Digitalwandler in ein digitales Signal umgewandelt und in der Auswerte- und Steuereinheit 11 verarbeitet und angezeigt. Die Auswerte- und Steuereinheit ist mit einer Alarmeinrichtung 12 verbunden, die ein Alarmsignal erzeugt, wenn Wasser im Öl festgestellt worden ist.

Der Mikrowellensender 8 ist als spannungsgesteuerter Osszilator ausgebildet, der von der Auswerte- und Steuereinheit 11 gesteuert wird. Die Frequenz wird nur in einem schmalbandigen Bereich geändert, und zwar um Verfälschungen durch Welligkeiten zu vermeiden. Die Auswerte und Steuereinheit 11 bildet in an sich bekannter Art und Weise den Mittelwert über die Einzelfrequenzen. Vorteilhafterweise wird die Frequenz der Mikrowellen im Bereich von 8 bis 24 GHz vorzugsweise 10 bis 13 GHz eingestellt.

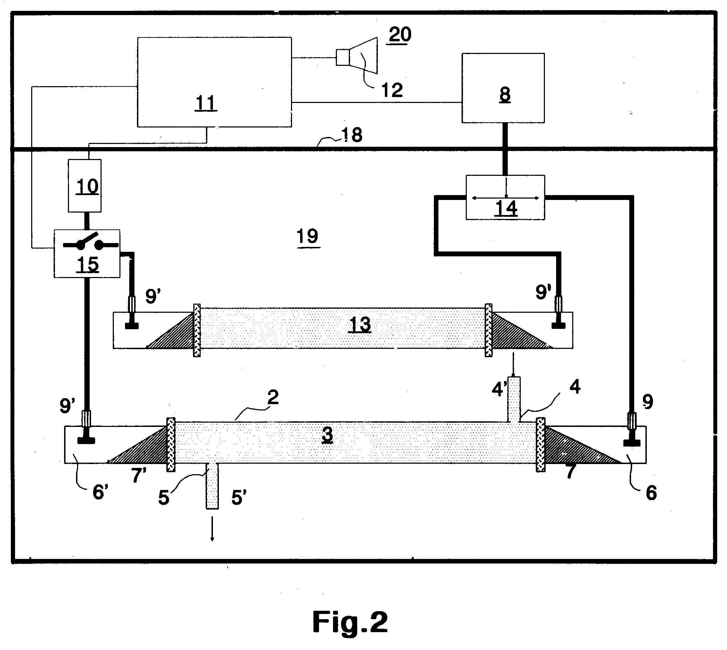

Eine zweite Ausführungsform ist in Fig. 2 dargestellt. Das Gerät beinhaltet eine Referenzmeßzelle 13, die analog zur Meßzelle 2 als metallischer Hohlleiter ausgebildet ist. Die Referenzmeßzelle 13 ist mit Öl gefüllt. Um die Mikrowellenleistung aufzuteilen, ist der Mikrowellensender mit einem Leistungsteiler 14 versehen. Über weitere Stiftkoppler 9' wird ein Teil der Mikrowellenleistung in die Referenzmeßzelle 13 ein- bzw. ausgekoppelt. Dem Detektor 10 ist ein Mikrowellenschalter 15 vorgeschaltet, der jeweils abwechselnd das Signal von der Meßzelle und das Referenzsignal von der Referenzmeßzelle 13 auf den Detektor 10 aufschaltet.

Schwankungen der Meßelektronik beispielsweise Frequenz oder Amplitudenschwankungen des Mikrowellensenders durch Alterungseffekte im Dauerbetrieb können mit Hilfe der Referenzmessung kompensiert werden. Durch Verhältnisbildung der beiden Detektorsignale entfallen sowie im Mikrowellensender als auch in der Auswerte- und Steuereinheit 11 Kurz- und Langzeitinstabilitäten, die sonst die Genauigkeit sowie die Auflösung erheblich verschlechtern können.

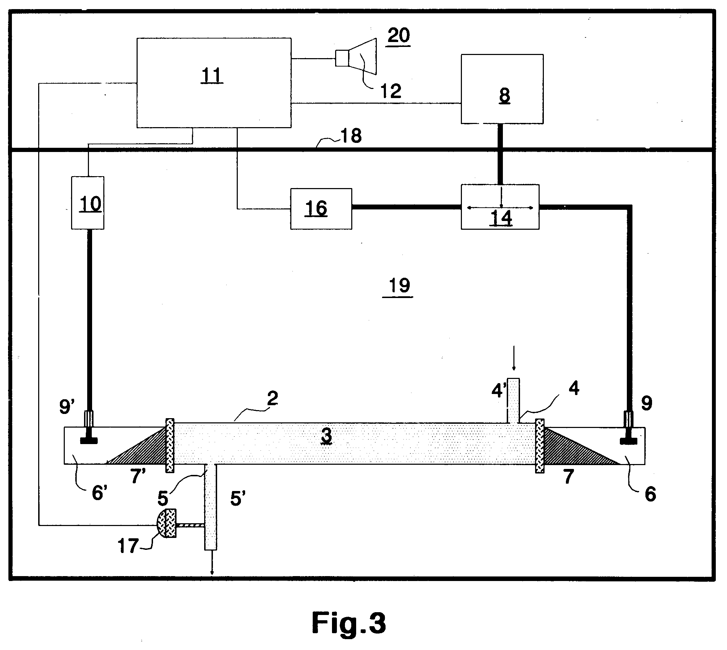

Die in Fig. 3 dargestellte Ausführungsform beinhaltet einen Referenzdetektor 16, der analog zum Detektor 10 ausgebildet ist. Über den Leistungsteiler 14 wird ein Teil der Mikrowellenleistung direkt in den Referenzdetektor 16 eingespeist. Das Referenzsignal von Referenzdetektor 16 wird zu der Auswerte- und Steuereinheit 11 geleitet und dort mit dem Signal aus der Meßzelle 2 verglichen.

Da die Temperatur des Öls schwanken kann, wird mit einem Widerstandsthermometer 17 eine Temperaturmessung durchgeführt. Das Meßergebnis wird zur Auswerte- und Steuereinheit 11 geleitet und zur Anpassung des Signals verwendet.

In den Fig. 1, 2 und 3 ist dargestellt, daß das Gehäuse 1 mittels einer Trennwand 18 in einen ersten Raum 19 und in einem zweiten Raum 20 geteilt ist. Im zweiten Raum 20 befinden sich die Auswerte- und Steuereinheit 11 sowie der Mikrowellensender 8. Die anderen Bauteile sind im ersten Raum 19 montiert. Auf diese Weise wird verhindert, daß durch einen eventuellen Austritt von Öl aus dem Meßraum 3 die elektronischen Bauteile beschädigt werden.

## Patentansprüche

1. Verfahren zur kontinuierlichen Messung des Wassergehaltes eines Fluides insbesondere von Öl in Ölbädern von Wärmebehandlungsanlagen, wobei von einem Mikrowellensender (8) erzeugte Mikrowellen durch das Fluid geleitet und von einem Detektor (10) empfangen werden und wobei der Detektor (10) ein zum Wassergehalt proportionales, analoges Signal abgibt, welches in einer nachgeschalteten Auswerte- und Steuereinheit (11) verarbeitet wird,
dadurch gekennzeichnet,
daß kontinuierlich ein Teilstrom des Fluides durch eine Meßzelle (2) in Form eines metallischen Hohlleiters geleitet wird und daß die Mikrowellen in den Hohlleiter eingekoppelt und ausgekoppelt werden.

2. Verfahren nach Anspruch 1,
dadurch gekennzeichnet,
daß die Frequenz der Mikrowellen im Bereich von 8 bis 24 GHz vorzugsweise 10 bis 13 GHz eingestellt wird.

3. Verfahren nach Anspruch 1 oder 2,
dadurch gekennzeichnet,
daß ein Teil der Mikrowellenleistung abgezweigt und zur Referenzmessung eingesetzt wird.

4. Verfahren nach einem der Ansprüche 1 bis 3,
dadurch gekennzeichnet,
daß das zum Wassergehalt proportionale Signal in Abhängigkeit von der Temperatur des Fluides angepaßt wird.

5. Gerät zur kontinuierlichen Messung des Wassergehaltes eines Fluides, insbesondere von Öl in Ölbädern von Wärmebehandlungsanlagen mit einer Meßzelle (2), einem Mikrowellensender (8) und einem Detektor (10), der mit einer Auswerte- und Steuereinheit (11) verbunden ist,
dadurch gekennzeichnet,
daß die Meßzeile (2) als metallischer Hohlleiter ausgebildet ist, daß die Meßzelle (2) einen Meßraum (3) aufweist, der mit mindestens einer Eintrittsöffnung (4) und mindestens einer Austrittsöffnung (5) für das Fluid versehen sind und daß sich an den Meßraum (3) stirnseitig je ein luftgefüllter Raum (6,6') anschließt, in den über je einen Stiftkoppler (9, 9') die Mikrowellen ein- bzw. ausgekoppelt werden.

6. Gerät nach Anspruch 5,
dadurch gekennzeichnet,
daß die Auswerte- und Steuereinheit (11) eine Alarmeinrichtung (12) beinhaltet, die ein Alarmsignal in Abhängigkeit vom Wassergehalt des Fluides erzeugt.

7. Gerät nach einem der Ansprüche 5 oder 6,
dadurch gekennzeichnet,
daß die Meßzelle (2) in Form eines Hohlleiters als im wesentlichen in sich geschlossener Rechteckhohlleiter ausgebildet ist.

8. Gerät nach einem der Ansprüche 5 bis 7,
dadurch gekennzeichnet,
daß die Eintrittsöffnung (4) und die Austrittsöffnung (5) an der breiten Seite des Rechteckhohlleiters angeordnet sind.

9. Gerät nach einem der Ansprüche 5 bis 8,
dadurch gekennzeichnet,
daß zwischen dem Meßraum (3) und dem luftgefüllten Raum (6,6') ein Anpaßelement (7,7') angeordnet ist.

10. Gerät nach einem der Ansprüche 5 bis 9,
dadurch gekennzeichnet,
daß das Anpaßelement (7,7') aus einem Material mit geringer Dielektrizitätskonstante, vorzugsweise Kunststoff besteht.

11. Gerät nach einem der Ansprüche 5 bis 10,
dadurch gekennzeichnet,
daß das Anpaßelement (7,7') keilförmig ist.

12. Gerät nach einem der Ansprüche 5 bis 11,
dadurch gekennzeichnet,
daß der Mikrowellensender (8) als spannungsgesteuerter Osszilator ausgebildet ist, der von der Auswerte- und Steuereinheit (11) gesteuert wird.

13. Gerät nach einem der Ansprüche 5 bis 12,
dadurch gekennzeichnet,
daß der Mikrowellensender (8) mit einem Leistungsteiler (14) versehen und dem Detektor (10), ein Mikrowellenschalter (15) vorgeschaltet ist, daß über einen weiteren Stiftkoppler (9') ein Teil der Mikrowellenleistung in eine Referenzmeßzelle (13) ein- bzw. ausgekoppelt werden und daß die Referenzmeßzelle (13) analog zur Meßzelle (2) als metallischer Hohlleiter ausgebildet ist.

14. Gerät nach einem der Ansprüche 5 bis 12,
dadurch gekennzeichnet,
daß der Mikrowellensender (8) mit einem Leistungsteiler (14) versehen ist, daß ein Teil der Mikrowellenleistung zu einem Referenzdetektor (16) geführt wird, und daß das Referenzsignal aus dem Referenzdetektor (16) zur Auswerte- und Steuereinheit (11) geführt wird.

15. Gerät nach einem der Ansprüche 5 bis 14,
dadurch gekennzeichnet,
ein Gehäuse (1), das mittels einer Trennwand (18) in zwei Räume (19, 20) geteilt ist, wobei in einem zweiten Raum (20) die Auswerte- und Steuereinheit (11) und der Mikrowellensender (8) und in dem ersten Raum (19) die übrigen Bauteile montiert sind.
